# EUROPÄISCHE PATENTANMELDUNG

(11) **EP 0 940 403 A2**
(43) Veröffentlichungstag der Anmeldung: **08.09.1999**
(21) Anmeldenummer: 99103506.4
(22) Anmeldetag: 24.02.1999
(51) Int. Cl.: C07D 487/22

(54) **Verfahren zur Herstellung von makropolycyclischen Polyaminen**

(30) Priorität: 05.03.1998 DE 19809543
(71) Anmelder: Clariant GmbH, 65929 Frankfurt am Main (DE)
(72) Erfinder: Nestler, Bernd, Dr., 65929 Frankfurt (DE); Seebach, Michael, Dr., 65795 Hattersheim (DE)

(57) **Zusammenfassung**

Beansprucht wird ein Verfahren zur Herstellung von makropolycyclischen Polyaminen der Formel 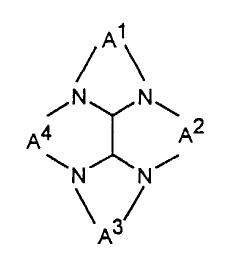 wobei A¹, A², A³ und A⁴ die in der Beschreibung genannte Bedeutung haben. Diese Verbindungen werden nach einem neuen, verbesserten Verfahren hergestellt durch Umsetzung eines cyclischen Amins der Formel 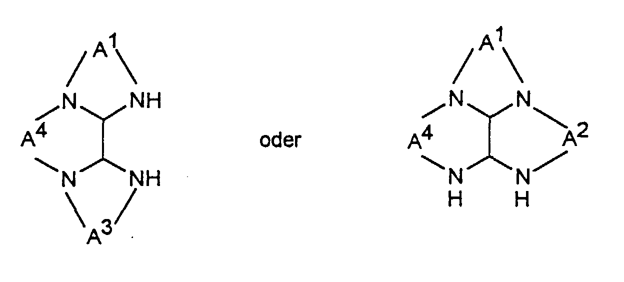 mit einer Verbindung der Formel X-A²-Y oder X-A³-Y, wobei A¹, A², A³ und A⁴ die in der Beschreibung angegebenen Bedeutungen haben und X und Y eine Abgangsgruppe bedeuten.

## Beschreibung

Diese Erfindung betrifft ein neues Verfahren zur Herstellung von speziellen makropolycyclischen Polyaminen mit einem Grundgerüst der allgemeinen Struktur (1) 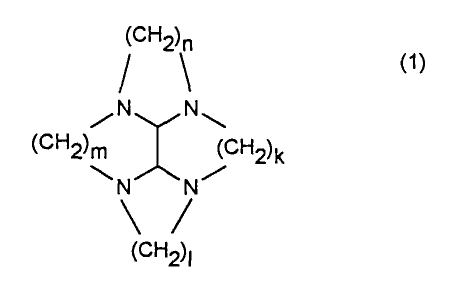 worin k, l, m und n unabhängig voneinander Zahlen von 2 bis 4 darstellen, die z. B. als Ausgangsmaterialien für die Herstellung weiterer cyclischer Polyaminverbindungen dienen können. Verwendbar sind derartige Polyaminverbindungen z. B. für Synthesen von pharmakologisch wirksamen Substanzen, von Liganden für katalytisch wirksame Metallkomplexe, von Host-Verbindungen für supramolekulare Strukturen oder von Protonenschwämmen.

Problematisch ist, daß die Ausgangsmaterialien für die Herstellung dieser makropolycyclischen Polyamine nur unter großem Aufwand und teilweise nur mit niedrigen Ausbeuten dargestellt werden können, da die bislang beschriebenen Methoden in aller Regel lediglich zur Synthese kleiner Mengen für wissenschaftliche Zwecke erarbeitet wurden.

Dies sei am Beispiel der Synthese von cis-Perhydro-3a,5a,8a,10a-tetraazapyren (2) (Formel (1) mit k, m = 2 und l, n = 3) illustriert. Hierfür sind folgende Verfahren bekannt.

Die Umsetzung von Cyclam (1,4,8,11-Tetraazacyclotetradecan) (3) mit Glyoxal in Acetonitril wurde von G. Weisman, E. Wong, D. Hill, M. Rogers, D. Reed und J. Calabrese in Chem. Commun. 1996, 947-948 beschrieben.

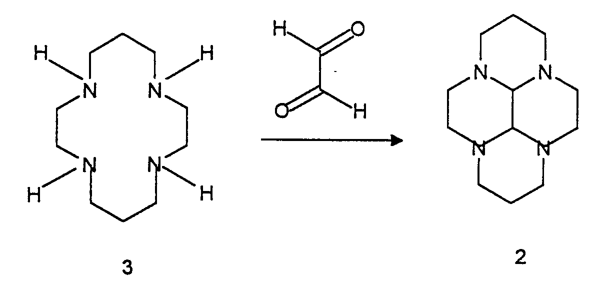

Hierbei wird cis-Perhydro-3a,5a,8a,10a-tetraazapyren in 80 % Ausbeute erhalten. Problematisch ist neben der Verwendung des giftigen Acetonitril als Solvens vor allem der Einsatz des nur schwer Zugänglichen Makrocyclus Cyclam als Ausgangsmaterial. Zur Cyclam-Herstellung sind folgende Verfahren beschrieben: WO 9708157 beschreibt ein dreistufiges Verfahren, in dem zunächst 1,3-Diaminopropan mit dem Chloracetylchlorid zum Bisamid I cyclisiert wird. Dieses wird im Folgeschritt mit 1,3-Diaminopropan in hochverdünnter Lösung zu 2,10-Dioxo-1,4,8,11-tetraazacyclotetradecan (II) cyclisiert und letzteres zu Cyclam reduziert:

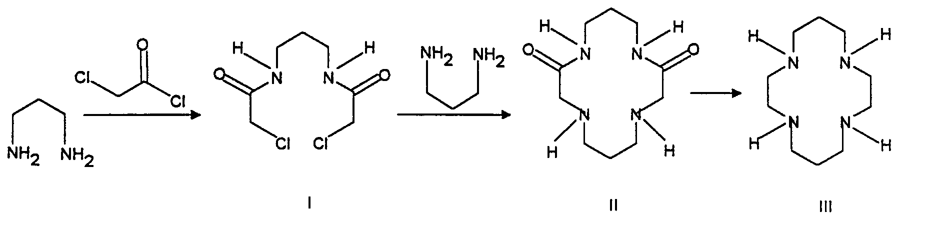

Als Reduktionsmittel wird dabei das nicht zuletzt aus Sicherheitsgründen nur schwer handhabbare Red-Al® eingesetzt; Ausbeuten werden für keinen der drei Reaktionsschritte genannt.
Ein anderes, mehrstufiges Verfahren zur Herstellung von Cyclam wird in WO 9705123 beschrieben. Hierbei wird 1,10-Diamino-4,7-diazadecan mit einem 4-5 fachen Überschuß Toluolsulfonsäurechlorid zu III umgesetzt, welches mit in einem getrennten Schritt hergestellten Ethylenglykolditosylat zum Tetratosylat IV cyclisiert und abschließend detosyliert wird.

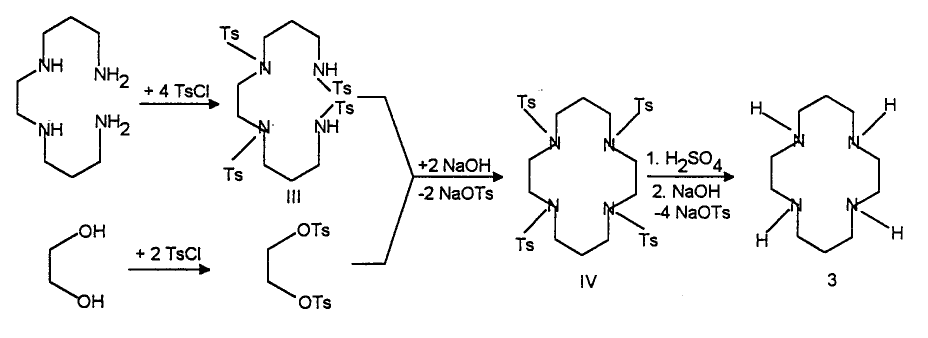

Nachteilig bei diesem Verfahren sind vor allem die benötigten Mengen an Ausgangsmaterialien und die gebildeten Mengen an Abfallstoffen. Pro Mol gebildetem Cyclam werden sechs Äquivalente Toluolsulfonsäurechlorid benötigt, entsprechend fallen sechs Mol Toluolsulfonsäure in Salzform als Abfall an.
Ein weiteres Herstellverfahren für Cyclam wird in EP 427595 aufgezeigt. Ausgehend von 1,2-Diaminoethan und Acrylnitril wird 1,9-Diamino-3,7-diazanonan synthetisiert. Zusatz von Nickel(II)chlorid ergibt den entsprechenden Nickel-Komplex, der mit Glyoxal in das Bisiminoderivat V überführt und katalytisch zum Nickel-Cyclamkomplex VI hydriert wird. Demetallierung durch Zusatz von Natriumcyanid ergibt Cyclam in 60 % Ausbeute. Kritisch bei dieser Templatsynthese ist 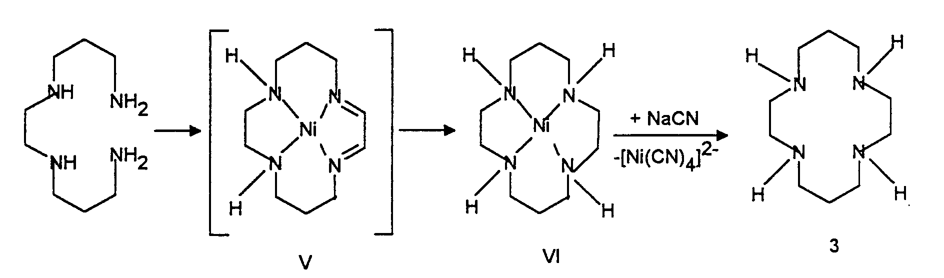 neben der Verwendung des allergieauslösenden Nickel vor allem das zur Dekomplexierung eingesetzte hochgiftige Cyanid und die Bildung von Nickelcyano-Verbindungen als Abfallprodukt.

Als weiterer Zugeng zu Perhydro-3a,5a,8a,10a-tetraazapyren wird von T. Okawara, H. Takaishi, Y. Okamoto, T. Yamasaki, M. Furukawa, in Heterocycles 41 (1995) 1023-1033 die Reduktion des durch sukzessive Umsetzung von 1,9-Diamino-3,7-diazanonan mit Glyoxal und Benzotriazol erhaltenen Dibenzotriazoylperhydrotetraazapyrens VII mit Natriumborhydrid genannt; entsprechend kann das aus 2,2-Bis(hexahydropyrimidin) resultierende Tetrabenzotriazoylperhydrotetraazapyren VIII 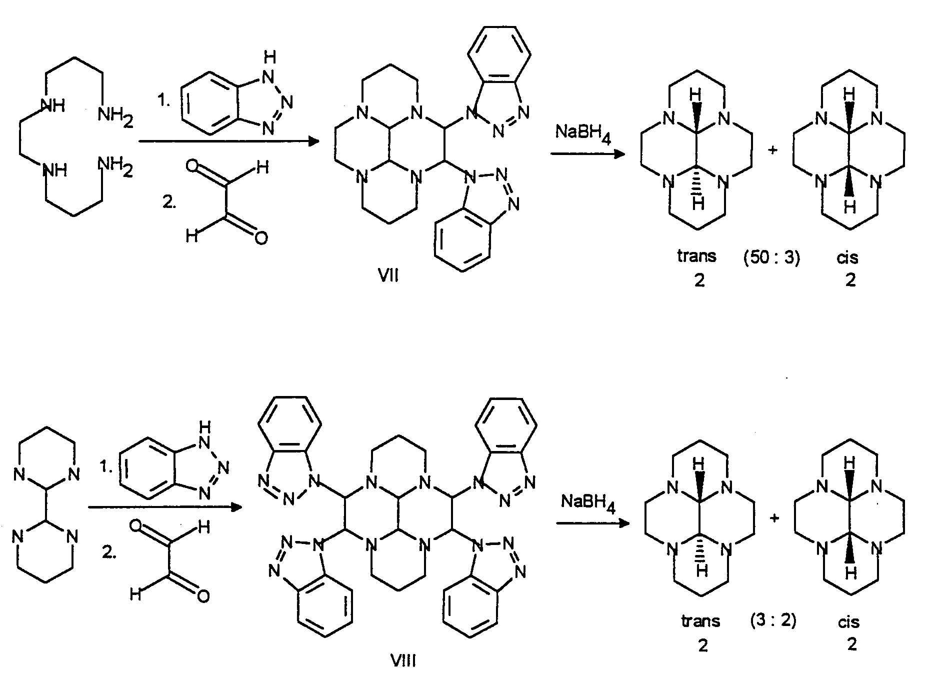 reduziert werden. Erhalten werden hierbei in jedem Fall Diastereomerengemische aus trans- und cis-Perhydro-3a,5a,8a,10a-tetraazapyren, wobei jeweils das trans-Isomer dominiert. Nachteilig bei diesem Verfahren ist die Verwendung von Benzotriazol, das abgespalten wird und als Abfall anfällt.

Diese bekannten Verfahren zur Herstellung der gewünschten polycyclischen Verbindungen mit einem Grundgerüst der allgemeinen Formel (**1**) sind somit aufgrund der Verwendung von zum Teil aufwendigen, vielstufigen Reaktionsführungen und schwer handhabbaren Reagentien zur Herstellung technischer Mengen dieser Makrocyclen ungeeignet.

Der Erfindung liegt die Aufgabe zugrunde, ein einfaches Verfahren zur Synthese von polycyclischen Verbindungen mit einem Grundgerüst der allgemeinen Formel (1) zu finden. Desweiteren soll das erfindungsgemäße Verfahren zur Herstellung technischer Mengen geeignet sein, wobei die entstehenden Produkte in hoher Ausbeute mit minimalen Abfallmengen anfallen sollen.

Gegenstand der Erfindung ist somit ein Verfahren zur Herstellung von polycyclischen Verbindungen der allgemeinen Formel (4) 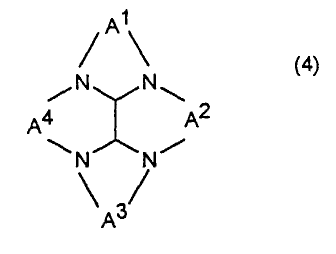 worin
A¹, A², A³, und A⁴ unabhängig voneinander entweder
   einen C₂- bis C₄-Alkylenrest, der durch eine oder mehrere Gruppen P und/oder Q substituiert sein kann, wobei
   P eine C₁- bis C₃₀-Alkyl- oder Cycloalkylgruppe, die mit einer oder mehreren Gruppen Q substituiert sein kann, und
   Q eine Gruppe des Typs
      - COR, worin R eine Hydroxy-, eine C₁- bis C₅ -Alkoxy- oder C₆- bis C₁₄-Aryloxy- oder eine substituierte oder unsubstituierte Aminogruppe,
      - eine C₆- bis C₁₄-Arylgruppe, die mit einer oder mehreren C₁- bis C₃₀-Alkyl-, Cycloalkyl-, Aryl-, C₁- bis C₅-Alkoxy-, C₆- bis C₁₄ -Aryloxy-, substituierten oder unsubstituierten Aminogruppen, Halogenatomen, Cyanogruppen, Sulfogruppen, Carboxylgruppen, oder Gruppierungen der Formel -(CH₂)ᵣ-COOH, -(CH₂)ᵣ-SO₃H, -(CH₂)ᵣ-PO₃H₂, -(CH₂)ᵣ OH, wobei r eine ganze Zahl von 0 bis 4 bedeutet und die genannten Säuregruppen auch in Salzform vorliegen können, substituiert sein kann,
      - ein aromatischer Heterocyclus, der Stickstoff-, Sauerstoff- und/oder Schwefelatome enthalten und der mit einer oder mehreren C₁- bis C₃₀-Alkyl-, Cycloalkyl-, Aryl-, C₁- bis C₅-Alkoxy-, C₆- bis C₁₄ -Aryloxy-, substituierten oder unsubstituierten Aminogruppen, Halogenatomen, Cyanogruppen, Sulfogruppen, Carboxylgruppen, oder Gruppierungen der Formel -(CH₂)ᵣ -COOH, -(CH₂)ᵣ-SO₃H, -(CH₂)ᵣ-PO₃H₂, -(CH₂)ᵣ OH, wobei r eine ganze Zahl von 0 bis 4 bedeutet und die genannten Säuregruppen auch in Salzform vorliegen können, substitiert sein kann,
      - eine substituierte oder unsubstituierte Aminogruppe,
      - eine Hydroxygruppe,
      - eine C₁-bis C₃₀-Alkyl- oder Cycloalkylgruppe,
      - eine C₁-bis C₃₀-Alkoxy- oder C₆- bis C₁₄-Aryloxygruppe,
      - ein Halogenatom,
      - eine Cyano-, Sulfo-, oder Carboxylgruppe,
      - eine Gruppierung der Formel -(CH₂)ᵣ -COOH, -(CH₂)ᵣ-SO₃H, -(CH₂)ᵣ-PO₃H₂, -(CH₂)ᵣ OH, wobei r eine ganze Zahl von 0 bis 4 bedeutet und die genannten Säuregruppen auch in Salzform vorliegen können;
oder A¹, A², A³ und A⁴ eine Gruppe -(CH₂)ₛ-E-(CH₂)ₜ-, worin E für einen C₆- bis C₁₄-Arylenrest, der mit Gruppen P und/oder Q substituiert sein kann, wobei den Gruppen P und Q die oben genannte Bedeutung zukommt und s und t unabhängig voneinander die Werte 0, 1 oder 2 einnehmen können, bedeutet.

Bevorzugt ist die Herstellung von Verbindungen der oben genannten Formel, wobei
A¹, A², A³ und A⁴ einen C₂-C₄-Alkylenrest bedeutet.

Dieses Verfahren besteht darin, daß man ein cyclisches Amin der Formel 5A oder 5B 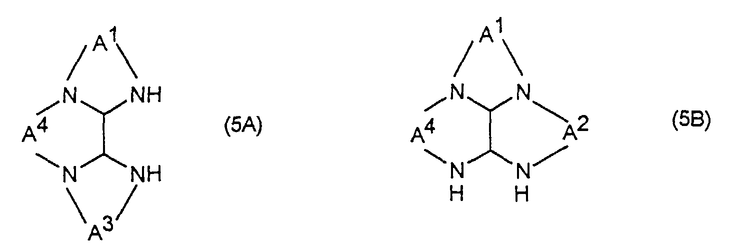 mit einer Verbindung der Formel

X-A²-Y (6A)

oder

X-A³-Y (6B)

umsetzt, wobei A¹, A², A³ und A⁴ in den Formeln 5 und 6 unabhängig voneinander die oben angegebenen Bedeutungen haben und X oder Y unabhängig voneinander für eine Abgangsgruppe stehen.

Die als Ausgangsstoffe für das erfindungsgemäße Verfahren dienenden cyclischen Amine der Formel 5A oder 5B können, wie z. B. von R. Müller, W. von Philipsborn, L. Schleifer, P. Aped und B. Fuchs in Tetrahedron 47 (1981) 1013-1036 beschrieben, durch Kondensation von Glyoxal mit Tetraaminen hergestellt werden. Beispiele für Grundgerüste derartiger cyclischer Amine sind cis- und trans-Octahydro-2a,5,6,8a-tetraazaacenaphtylen (aus 1,8-Diamino-3,6-diazaoctan), cis- und trans-Octahydro-1,3a,6a,9-tetraazaphenalen (aus 1,9-Diamino-3,7-diazanonan), cis- und trans-Dodecahydro-4,5,8a,10a-tetraazaphenantren (aus 1,10-Diamino-4-7-diazadecan), cis- und trans-Dodecahydro-1,4a,7a,11-tetraazadibenzo[ac]cyclohepten (aus 1,11-Diamino-4,8-diazaundecan).

Mit dem Begriff Abgangsgruppe sind solche Gruppen gemeint, die bei der Herstellung der Polyamine 4 unter den gewählten Reaktionsbedingungen aus den Verbindungen der Formel 6A bzw. 6B abgespalten werden, ohne daß diese durch andere Gruppen ersetzt werden. Hierzu zählen insbesondere die p-Toluol-, Benzol- und Methansulfonsäuregruppen, sowie die Halogenide Chlorid, Bromid und Iodid.

Die Umsetzung erfolgt üblicherweise in Gegenwart eines Lösungsmittels. Bevorzugte Lösungsmittel sind hierbei polare organische Lösungsmittel wie z. B. Alkohole, Glykole, Ether, Ketone, Ester, Carbonsäureamide, Halogenalkane und Sulfoxide oder Gemische derselben. Besonders bevorzugt sind Alkohole, Glykole und Carbonsäureamide mit kurzen und mittellangen Alkyl- oder Cycloalkylresten.

Die verwendeten Lösungsmittel können ohne weitere Reinigung oder Trocknung eingesetzt werden. Lediglich wenn die im Lösungsmittel vorhandene Wassermenge 3 - 5 % übersteigt, sinken die Ausbeuten merklich ab.

Üblicherweise wird für die Reaktion soviel Lösungsmittel eingesetzt, daß sich die Verbindungen 5A bzw. 5B und 6A bzw. 6B darin lösen. Sollte keine vollständige Lösung erfolgen, kann auch in Dispersion (Suspension bzw. Emulsion) gearbeitet werden. Die Konzentration an Verbindungen der Formel 5A bzw. 5B und 6A bzw. 6B liegt üblicherweise im Bereich von 0.01 bis 2.5 mol pro Liter Lösungsmittel, bevorzugt 0.05 bis 1.2 mol/l, besonders bevorzugt 0.1 bis 0.7 mol/l.

Die erfindungsgemäße Umsetzung kann vorteilhafterweise in Gegenwart von einer oder mehreren Basen durchgeführt werden. Als Basen geeignet sind anorganische oder organische Basen. Beispielhaft hierfür seien genannt: Metallhydride, -amide, -hydroxide, -carbonate, -hydrogencarbonate, -phosphate, -hydrogenphosphate, und/oder organische Amine. Bevorzugt verwendet werden Alkalimetallcarbonate sowie Amine. Im allgemeinen wird pro Äquivalent abzuspaltendem Proton des sekundären Stickstoffatoms der Verbindungen der Formel 5A bzw. 5B 0.8 bis 10.0 Moläquivalente, bevorzugt 0.9 bis 5.0 Moläquivalente, besonders bevorzugt 1.0 bis 2.5 Moläquivalente Base verwendet. Die Base kann fest oder in den vorstehend genannten Lösungsmitteln gelöst oder suspendiert eingebracht werden. Beispiele für feste Basen sind Pulver, Schuppen, Microprills oder Plätzchen.

Die Reaktion erfolgt bei Temperaturen zwischen 10 bis 200°C, bevorzugt 50 bis 150°C. Der hierfür benötigte Zeitraum beträgt, in Abhängigkeit von der gewählten Temperatur etwa 1 Stunde bis 8 Tage. Da eine Erhöhung der Umsetzungstemperatur im allgemeinen in einer Verkürzung der Reaktionszeit resultiert, ist es vorteilhaft, die Umsetzung bei erhöhter Temperatur, ggf. in der Nähe der Siedetemperatur des Lösungsmittels, durchzuführen.

Zur Durchführung des erfindungsgemäßen Verfahrens wird im allgemeinen ein Gemisch aus den Verbindungen der Formeln 5A bzw. 5B und 6A bzw. 6B, dem Lösungsmittel und ggf. der Base auf die Umsetzungstemperatur erhitzt. Dabei ist es unerheblich, in welcher Reihenfolge die einzelnen Bestandteile des Reaktionsgemisches zusammen-gegeben werden.
Nach Beendigung der Reaktion wird - soweit erforderlich - die gebildete Zielverbindung aus dem Reaktionsgemisch mit den üblichen Verfahren isoliert. Geeignete Verfahren sind z. B. Filtration, Extraktion, destillative, chromatographische und osmotische Verfahren, sowie Kombinationen dieser Verfahren. Zur Vermeidung von Nebenreaktionen erfolgt die Umsetzung und ggf. die Aufarbeitung unter Schutzgas, üblicherweise Stickstoff.

Mit Hilfe des erfindungsgemäßen Verfahrens werden bevorzugt polycyclische Polyamine mit einem Grundgerüst wie
cis- und trans-Decahydro-2a,4a,6a,8a-tetraazacyclopenta[fg]acenaphtylen,
cis- und trans-Decahydro-2a,4a,7a,9a-tetraazacyclopenta[cd]phenalen,
cis- und trans-Decahydro-3a,5a,8a,10a-tetraazapyren,
cis- und trans-Decahydro-3a,5a,8a,11a-tetraazacyclohepta[def]phenantren oder
cis- und trans-Decahydro-3a,6a,9a,12a-tetraazadibenzo[ef,kl]heptalen hergestellt.

Das erfindungsgemäße Verfahren zeichnet sich insbesondere durch folgende Vorteile aus:
- Geringe Abfallmengen
- Sehr gute Ausbeuten
- Einfach zugängliche, z. T. kommerziell auch in großer Menge verfügbare Ausgangsstoffe
- z. T. Kontrolle des Isomerenverhältnisses des Produktes durch Wahl der Reaktionsbedingungen.

Die folgenden, für die Reaktionsführung typischen Beispiele dienen der näheren Erläuterung des erfindungsgemäßen Verfahrens und stellen keine Einschränkung der Anwendbarkeit des Verfahrens dar.

### Beispiel 1: Synthese von cis-Octahydro-1,3a,6a,9-tetraazaphenalen

Zu einer Lösung von 8.01 g 1,9-Diamino-3,7-diazanonan in 30 ml Ethanol wurden unter Kühlung innerhalb von 15 Minuten eine Lösung von 7.26 g einer 40 % wäßrigen Glyoxallösung zugetropft. Nach einstündigem Stehenlassen wurde zur Trockene eingedampft. Erhalten wurden 18.2 g eines hellgelben Feststoffs.

### Beispiel 2: Synthese von trans-Dodecahydro-4,5,8a,10a-tetraazaphenanthren

Eine Lösung von 87.2 g 1,10-Diamino-4,7-diazadecan in 500 ml Toluol wurde mit 72.3 g einer 40 % wäßrigen Glyoxallösung versetzt und das Gemisch 3 Stunden mit aufgesetztem Wasserabscheider refluxiert. Beim Abkühlen der Lösung fiel ein Feststoff aus, dieser wurde abgesaugt, dreimal mit jeweils 50 ml Toluol gewaschen und im Vakuum getrocknet. Man erhielt 34.9 g schwach gelbe Nadeln.

### Beispiel 3: Synthese von cis-Decahydro-3a,5a,8a,10a-tetraazapyren

Ein Gemisch aus 9.11 g cis-Octahydro-1,3a,6a,9-tetraazaphenalen, 19.2 g 1,3-Propandiol-di-(p-toluolsulfonat), 6.91 g Kaliumcarbonat und 50 ml trockenem Ethanol wurde unter Rückfluß 8 Stunden zum Sieden erhitzt. Das Reaktionsgemisch wurde filtriert, das Filtrat zur Trockene eingedampft und das Produkt aus dem so erhaltenen Rückstand mit 50 ml Methylenchlorid extrahiert. Entfernen des Solvens am Rotationsverdampfer ergab 8.22 g gelbbraunes Öl; Destillation im Vakuum ergab ein farbloses Produkt.

### Beispiel 4: Synthese von cis-Decahydro-3a,5a,8a,10a-tetraazapyren

Cis-Decahydro-3a,5a,8a,10a-tetraazapyren wurde wie in Beispiel 3 beschrieben synthetisiert; dabei wurde in der Reaktion 1,3-Dibrompropan anstelle von Propandiol-di-(p-toluolsulfonat) umgesetzt.

### Beispiel 5: Synthese von trans-Decahydro-3a,5a,8a,10a-tetraazapyren

Trans-Decahydro-3a,5a,8a,10a-tetraazapyren wurde wie in Beispiel 3 beschrieben synthetisiert; dabei wurde Triethylamin anstelle von Kaliumcarbonat eingesetzt.

### Beispiel 6: Synthese von trans-Decahydro-3a,5a,8a,10a-tetraazapyren

Trans-Decahydro-3a,5a,8a,10a-tetraazapyren wurde wie in Beispiel 3 beschrieben synthetisiert; dabei wurde in der Umsetzung Pyridin anstelle von Kaliumcarbonat eingesetzt.

### Beispiel 7: Synthese von trans-Decahydro-3a,5a,8a,10a-tetraazapyren

Ein Gemisch aus 5.90 g trans-Dodecahydro-4,5,8a,10a-tetraazaphenanthren, 11.1 g 1,2-Ethandiol-di-(p-toluolsulfonat), 4.20 g Kaliumcarbonat und 80 ml trockenem N-Methylpyrrolidon wurde 2.5 Stunden auf 130 °C und anschließend 6 Stunden auf 180 °C erhitzt. Das Reaktionsgemisch wurde zur Trockene eingedampft und das Produkt aus dem so erhaltenen Rückstand mit 50 ml Methylenchlorid extrahiert. Entfernen des Solvens am Rotationsverdampfer ergab 6.52 g gelbbraunes Öl.

## Patentansprüche

1. Verfahren zur Herstellung von polycyclischen Verbindungen der allgemeinen Formel 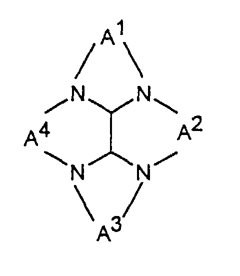 worin
A¹, A², A³, und A⁴ unabhängig voneinander entweder
einen C₂- bis C₄-Alkylenrest, der durch eine oder mehrere Gruppen P und/oder Q substituiert sein kann, wobei
P eine C₁- bis C₃₀-Alkyl- oder Cycloalkylgruppe, die mit einer oder mehreren Gruppen Q substituiert sein kann, und
Q eine Gruppe des Typs
- COR, worin R eine Hydroxy-, eine C₁- bis C₅ -Alkoxy- oder C₆- bis C₁₄- Aryloxy- oder eine substituierte oder unsubstituierte Aminogruppe,
- eine C₆- bis C₁₄-Arylgruppe, die mit einer oder mehreren C₁- bis C₃₀-Alkyl-, Cycloalkyl-, Aryl-, C₁- bis C₅-Alkoxy-, C₆- bis C₁₄ -Aryloxy-, substituierten oder unsubstituierten Aminogruppen, Halogenatomen, Cyanogruppen, Sulfogruppen, Carboxylgruppen, oder Gruppierungen der Formel -(CH₂)ᵣ-COOH, -(CH₂)ᵣ-SO₃H, -(CH₂)ᵣ-PO₃H₂, -(CH₂)ᵣ OH, wobei r eine ganze Zahl von 0 bis 4 bedeutet und die genannten Säuregruppen auch in Salzform vorliegen können, substituiert sein kann,
- ein aromatischer Heterocyclus, der Stickstoff-, Sauerstoff- und/oder Schwefelatome enthalten und der mit einer oder mehreren C₁- bis C₃₀-Alkyl-, Cycloalkyl-, Aryl-, C₁- bis C₅-Alkoxy-, C₆- bis C₁₄ -Aryloxy-, substituierten oder unsubstituierten Aminogruppen, Halogenatomen, Cyanogruppen, Sulfogruppen, Carboxylgruppen, oder Gruppierungen der Formel -(CH₂)ᵣ -COOH, -(CH₂)ᵣ-SO₃H, -(CH₂)ᵣ-PO₃H₂, -(CH₂)ᵣ OH, wobei r eine ganze Zahl von 0 bis 4 bedeutet und die genannten Säuregruppen auch in Salzform vorliegen können, substitiert sein kann,
- eine substituierte oder unsubstituierte Aminogruppe,
- eine Hydroxygruppe,
- eine C₁-bis C₃₀-Alkyl- oder Cycloalkylgruppe,
- eine C₁-bis C₃₀-Alkoxy- oder C₆- bis C₁₄-Aryloxygruppe,
- ein Halogenatom,
- eine Cyano-, Sulfo-, oder Carboxylgruppe,
- eine Gruppierung der Formel -(CH₂)ᵣ -COOH, -(CH₂)ᵣ-SO₃H, -(CH₂)ᵣ-PO₃H₂, -(CH₂)ᵣ OH, wobei r eine ganze Zahl von 0 bis 4 bedeutet und die genannten Säuregruppen auch in Salzform vorliegen können;
oder A¹, A², A³ und A⁴ eine Gruppe -(CH₂)ₛ-E-(CH₂)ₜ-, worin E für einen C₆- bis C₁₄-Arylenrest, der mit Gruppen P und/oder Q substituiert sein kann, wobei den Gruppen P und Q die oben genannte Bedeutung zukommt und s und t unabhängig voneinander die Werte 0, 1 oder 2 einnehmen können, bedeutet, dadurch gekennzeichnet, daß man ein cyclisches Amin der Formel 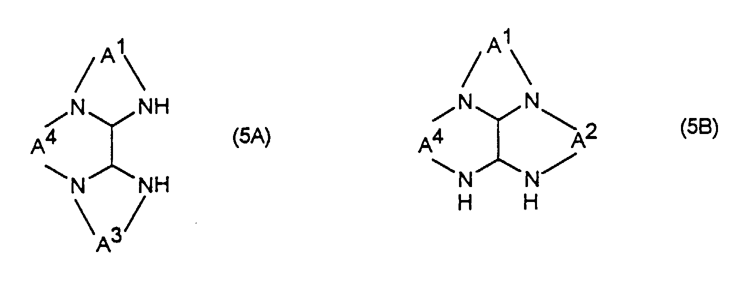 mit einer Verbindung der Formel
X-A²-Y (6A)
oder
X-A³-Y (6B)
umsetzt, wobei A¹, A², A³ und A⁴ unabhängig voneinander die oben für angegebenen Bedeutungen haben und X oder Y unabhängig voneinander für eine Abgangsgruppe stehen.

2. Verfahren nach Anspruch 1, dadurch gekennzeichnet, daß man Verbindungen der dortigen Formel herstellt, wobei A¹, A², A³ und A⁴ unabhängig voneinander einen C₂-C₄-Alkylenrest bedeuten.

3. Verfahren nach Anspruch 1, dadurch gekennzeichnet, daß man die Umsetzung in Gegenwart eines polaren organischen Lösungsmittel durchführt.

4. Verfahren nach Anspruch 1, dadurch gekennzeichnet, daß man die Umsetzung in Gegenwart einer Base durchführt.

5. Verfahren nach Anspruch 1, dadurch gekennzeichnet, daß man die Umsetzung bei Temperaturen 10 bis 200 °C durchführt.
